# EUROPEAN PATENT APPLICATION

(11) **EP 1 321 137 A1**
(43) Date of publication of application: **25.06.2003**
(21) Application number: 02258819.8
(22) Date of filing: 20.12.2002
(51) Int. Cl.: A61K 9/08, A61K 9/70

(54) **Film forming liquid composition**

(30) Priority: 21.12.2001 US 29614
(71) Applicant: Johnson & Johnson Consumer Companies, Inc., Skillman, New Jersey 08558-9418 (US)
(72) Inventor: Kundel, Nikhil, Piscataway, New Jersey 08854 (US)
(74) Representative: Mercer, Christopher Paul

(57) **Abstract**

A composition comprising: a film forming polymer; a low boiling point solvent; a high boiling point solvent; and at least one active ingredient is disclosed. The composition is useful for protecting damaged skin and delivering active ingredients to the skin.

## Description

### Background of the Invention

### Field of the Invention

The present invention relates to a film forming liquid composition. The liquid composition includes a film forming polymer, a low boiling point solvent, and a high boiling point solvent. Active ingredients, such as anti-itch actives, cooling actives, antibiotics, and the like are included in the compositions. The compositions are applied to the skin and the low boiling point solvent evaporates, leaving a polymeric film on the skin. The film which is left on the skin is plasticized by the high boiling point solvent included in the liquid composition. The polymeric film delivers the active ingredients to the skin over time.

### Description of the Prior Art

Minor cuts and scrapes, sunburn, bug bites, and poison ivy are conditions that cause discomfort for many consumers. These conditions frequently cause the skin to feel itchy. Consumers tend to scratch the itch. Scratching the itchy area can result in further damage to the skin, including infection. Therefore, health care professionals recommend keeping the affected area clean and dry.

Many anti-itch products are commercially available. The anti-itch products that are commercially available tend to be in the form of liquid sprays or lotions that are sprayed onto or rubbed into the affected area of the skin. The liquid sprays tend to have a viscosity similar to that of water. This means that the liquid tends to spread too easily, can drip off the skin onto which it has been sprayed, and can be messy with respect to articles of clothing.

The commercially available anti-itch lotions tend to be much more viscous than the liquid sprays. The lotions may be difficult to pour and to spread on the skin. Frequently, the lotions are pink, due to the color of the active ingredient present therein. This can be unsightly, as the area of skin where the lotion is applied becomes pink. The pink color is also problematic for staining articles of clothing.

Gel compositions have been developed for treating sunburn and the itch associated with poison ivy. These gel compositions utilize clays to form the gel. The gels tend to be highly viscous. The high viscosity is desirable, as it prevents the composition from running or dripping. The gels are typically sold in squeeze tubes.

Although liquid anti-itch products are effective, consumers frequently scratch the area of the skin to which the anti-itch product has been applied. It would be advantageous to provide a composition that provides a barrier, which discourages the consumer from scratching the affected area of skin and delivers active ingredients to the skin.

A product sold under the designation "New Skin" has been commercially available for quite some time. This product contains nitrocellulose in acetone. The product is used to cover damaged skin. It is quite common to see a bowler develop a blister on the thumb used for bowling. The "New Skin" product is frequently applied to the affected area to protect the skin from further damage. Alternatively, the "New Skin" product may be applied to the skin to prevent damage in the first instance. The "New Skin" composition does not contain an active ingredient or a plasticizer.

There is a continuing need for a composition that provides a barrier to scratching the skin and delivers active ingredients to the skin.

### Summary of the Invention

In accordance with one aspect of the present invention, there is provided a film forming liquid composition for use in delivering actives to skin and protecting the skin. The liquid composition includes a film forming polymer; a low boiling point solvent; a high boiling point solvent, and at least one active ingredient.

In a second embodiment, the present invention provides a method for protecting skin and delivering at least one active ingredient to the skin including applying a composition having a film forming polymer; a low boiling point solvent; a high boiling point solvent, and at least one active ingredient to the damaged skin.

### Detailed Description of Preferred Embodiments

The compositions of the present invention include a film forming polymer. The film forming polymer is suitable for contact with damaged skin. Suitable film forming polymers include, but are not limited to, cellulose acetate, cellulose acetate butyrate, cellulose acetate propionate, and ethylene vinyl acetate. Copolymers and blends of polymers may also be used in the practice of the present invention. Cellulose acetate butyrate is the presently preferred film-forming material. The amount of film forming polymer utilized in the compositions of the present invention may range from about 3 percent to about 15 percent, preferably from about 3 percent to about 10 percent by weight, based on the total weight of the composition.

The compositions of the present invention also include a low boiling point solvent. Suitable low boiling point solvents have boiling points below about 100° F. Suitable low boiling point solvents include, but are not limited to, n-hexane, n-heptane, n-octane, cyclohexane, cyclopentane, methanol, ethanol, n-propanol, isopropanol, n-butyl alcohol, methyl ethyl ketone, ethyl acetate, and acetone. Ethyl acetate is preferred as the low boiling point solvent. The amount of low boiling point solvent in the compositions of the present invention may range from about 70 percent to about 95 percent, preferably from about 75 percent to about 95 percent by weight, based on the total weight of the composition.

A high boiling point solvent is included in the compositions of the present invention to plasticize the film forming polymer. Suitable high boiling point solvents have boiling points above about 100° F. Suitable high boiling point solvents include, but are not limited to, triacetin, tributyrin, triethyl citrate, and combinations thereof. The amount of high boiling point solvent may range from about 1 percent to about 10 percent by weight, preferably from about 1 percent to about 5 percent by weight, based on the total weight of the composition.

The compositions of the present invention include at least one active ingredient for delivery to skin. Typical actives include pain relief active ingredients; itch relief active ingredients; antibiotics; antifungal agents; antihistamine agents; anti-inflammatory agents; antipruritic agents; skin and mucous membrane agents; wound care agents; and combinations thereof. Specific active ingredients include, but are not limited to, benzocaine, menthol, camphor, and diphenhydramine. Menthol, camphor, benzocaine, and combinations thereof are preferred. As is known in the art, the amount of active ingredient may vary, depending on the desired effect. Generally, the total amount of active ingredient may range from about 0.05% to about 30% by weight, based on the total weight of the composition.

The compositions of the present invention are prepared by admixing the ingredients in a suitable vessel and stirring the mixture. The compositions are applied to the affected area of the skin. The low boiling point solvent evaporates, leaving the plasticized polymer in film form on the skin. The active ingredient is released from the film over time and is thereby delivered to the skin.

Several examples are described below to illustrate the present invention. The invention should not be construed as being limited to the details thereof.

### Example 1

The following materials were combined in a vessel:
149.64 g ethyl acetate (low boiling point solvent),
20 g cellulose acetate butyrate (film forming polymer), and
20 g triacetin (high boiling point solvent).
The solution was stirred until the polymer was dissolved and the solution became clear. The following ingredients were then added with continued stirring:
10 g benzocaine (topical anesthetic),
0.18 g menthol, and
0.18 g camphor.
The solution was stirred until it became clear again. The composition contained 10% cellulose acetate butyrate, 0.09% menthol, 0.09% camphor, 10% triacetin, 5% benzocaine, and 74.82% ethyl acetate.

### Example 2

The following materials were combined in a vessel:
149.64 g ethyl acetate (low boiling point solvent),
20 g cellulose acetate butyrate (film forming polymer), and
20 g tributyrin (high boiling point solvent).
The solution was stirred until the polymer was dissolved and the solution became clear. The following ingredients were then added with continued stirring:
10 g benzocaine,
0.18 g menthol, and
0.18 g camphor.
The solution was stirred until it became clear again. The composition contained 10% cellulose acetate butyrate, 0.09% menthol, 0.09% camphor, 10% tributyrin, 5% benzocaine, and 74.82% ethyl acetate.

### Example 3

The following materials were combined in a vessel:
149.64 g ethyl acetate (low boiling point solvent),
20 g cellulose acetate butyrate (film forming polymer), and
20 g Triethyl citrate (high boiling point solvent).
The solution was stirred until the polymer was dissolved and the solution became clear. The following ingredients were then added with continued stirring:
10 g benzocaine,
0.18 g menthol, and
0.18 g camphor.
The solution was stirred until it became clear again. The composition contained 10% cellulose acetate butyrate, 0.09% menthol, 0.09% camphor, 10% Triethyl citrate, 5% benzocaine, and 74.82% ethyl acetate.

### Example 4

The following materials were combined in a vessel:
186 g ethyl acetate (low boiling point solvent),
10 g cellulose acetate butyrate (film forming polymer), and
3.8 g triacetin.
The solution was stirred until the polymer was dissolved and the solution became clear. The following ingredient was then added with continued stirring:
0.2 g camphor.
The solution was stirred until it became clear again. The composition contained 5% cellulose acetate butyrate, 0.1% camphor, 1.9% triacetin, and 93% ethyl acetate.

### Example 5

The following materials were combined in a vessel:
186 g ethyl acetate (low boiling point solvent),
10 g cellulose acetate butyrate (film forming polymer), and
3.8 g triacetin.
The solution was stirred until the polymer was dissolved and the solution became clear. The following ingredient was then added with continued stirring:
0.2 g menthol.
The solution was stirred until it became clear again. The composition contained 5% cellulose acetate butyrate, 0.1% menthol, 1.9% triacetin, and 93% ethyl acetate.

### Example 6

The following materials were combined in a vessel:
185 g ethyl acetate (low boiling point solvent),
10 g cellulose acetate butyrate (film forming polymer), and
4 g triacetin.
The solution was stirred until the polymer was dissolved and the solution became clear. The following ingredient was then added with continued stirring:
1 g camphor.
The solution was stirred until it became clear again. The composition contained 5% cellulose acetate butyrate, 0.5% camphor, 2% triacetin, and 92.5% ethyl acetate.

### Example 7

The following materials were combined in a vessel:
185 g ethyl acetate (low boiling point solvent),
10 g cellulose acetate butyrate (film forming polymer), and
4 g triacetin.
The solution was stirred until the polymer was dissolved and the solution became clear. The following ingredient was then added with continued stirring:
1 g menthol.
The solution was stirred until it became clear again. The composition contained 5% cellulose acetate butyrate, 0.5% menthol, 2% triacetin, and 92.5% ethyl acetate.

### Example 8

The following materials were combined in a vessel:
181 g ethyl acetate (low boiling point solvent),
12 g cellulose acetate butyrate (film forming polymer), and
6 g triacetin.
The solution was stirred until the polymer was dissolved and the solution became clear. The following ingredient was then added with continued stirring:
1 g menthol.
The solution was stirred until it became clear again. The composition contained 6% cellulose acetate butyrate, 0.5% menthol, 3% triacetin, and 90.5% ethyl acetate.

### Example 9

The following materials were combined in a vessel:
185 g ethyl acetate (low boiling point solvent),
10 g cellulose acetate butyrate (film forming polymer), and
4 g tributyrin.
The solution was stirred until the polymer was dissolved and the solution became clear. The following ingredient was then added with continued stirring:
1 g menthol.
The solution was stirred until it became clear again. The composition contained 5% cellulose acetate butyrate, 0.5% menthol, 2% tributyrin, and 92.5% ethyl acetate.

### Example 10

The following materials were combined in a vessel:
180.2 g ethyl acetate (low boiling point solvent),
12 g cellulose acetate butyrate (film forming polymer), and
6 g tributyrin.
The solution was stirred until the polymer was dissolved and the solution became clear. The following ingredient was then added with continued stirring:
1.8 g menthol.
The solution was stirred until it became clear again. The composition contained 6% cellulose acetate butyrate, 0.9% menthol, 3% tributyrin, and 90.1% ethyl acetate.

### Example 11

The following materials were combined in a vessel:
180.2 g ethyl acetate (low boiling point solvent),
10 g cellulose acetate butyrate (film forming polymer), and
4 g triethyl citrate.
The solution was stirred until the polymer was dissolved and the solution became clear. The following ingredient was then added with continued stirring:
1.8 g menthol.
The solution was stirred until it became clear again. The composition contained 5% cellulose acetate butyrate, 0.9% menthol, 2% triethyl citrate, and 90.1% ethyl acetate.

### Example 12

The following materials were combined in a vessel:
182 g ethyl acetate (low boiling point solvent),
10 g cellulose acetate butyrate (film forming polymer), and
6 g triacetin.
The solution was stirred until the polymer was dissolved and the solution became clear. The following ingredient was then added with continued stirring:
2 g camphor.
The solution was stirred until it became clear again. The composition contained 5% cellulose acetate butyrate, 1% camphor, 3% triacetin, and 91% ethyl acetate.

In the foregoing Examples, benzocaine functions as a topical anesthetic; and camphor and methol function as anti-itch agents.

The compositions of Examples 1-12 above were tested by brushing approximately one ml of each composition on the skin of a finger to cover approximately a 1 cm x 2.5 cm area of skin. The compositions were evaluated for drying time, water resistance, and film quality.

The compositions all dried within 1 minute and formed clear, almost invisible films. The polymer film coated fingers were placed under hot (approximately 50°C) and cold (approximately 25°C) water running from a tap. The polymer films were water resistant and adhered to the skin even under the running water. The polymer films had very little tack, and therefore did not pick up any dirt. The films adhered well to the skin for more than eight hours with excellent bioadhesion (the films did not curl up at the edges).

Based on the above results, the compositions of the present invention provide an excellent barrier over damaged skin to prevent scratching and further damage. The films also discourage the consumer from scratching the affected area of skin and function to deliver active ingredients to the skin.

## Claims

1. A composition comprising:
a film forming polymer;
a low boiling point solvent;
a high boiling point solvent, and
at least one active ingredient.

2. The composition according to claim 1 wherein the film forming polymer is cellulose acetate, cellulose acetate butyrate, cellulose acetate propionate or ethylene vinyl acetate.

3. The composition according to claim 1 or claim 2 wherein the low boiling point solvent is n-hexane, n-heptane, n-octane, cyclohexane, cyclopentane, methanol,ethanol, n-propanol, isopropanol, n-butyl alcohol, methyl ethyl ketone, ethyl acetate or acetone.

4. The composition according to any one of claims 1 to 3 wherein the high boiling point solvent is triacetin, tributyrin, triethyl citrate or a combination thereof.

5. The composition according to any one of claims 1 to 4 wherein the active ingredient is a pain relief active ingredient; an itch relief active ingredient; an antibiotic; an antifungal agent; an antihistamine agent; an anti-inflammatory agent; an antipruritic agent; a skin or mucous membrane agent; a wound care agent; or a combination thereof.

6. The composition according to claim 1 wherein the amount of film forming polymer ranges from 3 percent to 15 percent, preferably from 3 percent to 10 percent, the amount of the low boiling point solvent ranges from 70 percent to 95 percent, preferably from 75 percent to 95 percent, the amount of the high boiling point solvent ranges from 1 percent to 10 percent, preferably from 1 percent to 5 percent, and the amount of active ingredient ranges from 0.05% to 30% by weight, based on the total weight of the composition.

7. The composition according to claim 6 wherein the film forming polymer is cellulose acetate butyrate, the low boiling point solvent is ethyl acetate, and the high boiling point solvent is triacetin.

8. The composition according to claim 7 wherein the active ingredient is a combination of camphor, menthol and benzocaine.

9. The composition according to claim 8 wherein the amount of camphor ranges from 0.1% to 3%, the amount of menthol ranges from 0.1% to 1%, and the amount of benzocaine ranges from 5% to 20% by weight, based on the total weight of the composition.

10. The composition of any one of claims 1 to 9 for protecting damaged skin and delivering at least one active ingredient to the skin.
